# EUROPEAN PATENT SPECIFICATION

(11) **EP 4 331 497 B1**
(45) Date of publication and mention of the grant of the patent: **14.05.2025**
(21) Application number: 22192554.8
(22) Date of filing: 29.08.2022
(51) Int. Cl.: A61B 6/51, A61B 6/00, A61B 6/40

(54) **X-RAY SYSTEM FOR THE PRODUCTION OF DENTAL PANORAMIC IMAGES WITH SEVERAL OFFSET INDIVIDUAL X-RAY EMITTERS**
RÖNTGENSYSTEM ZUR PRODUKTION VON ZAHNMEDIZINISCHEN PANORAMABILDERN MIT MEHREREN VERSETZTEN EINZELSTRAHLERN
SYSTÈME À RAYONS X POUR LA PRODUCTION D'IMAGES PANORAMIQUES DENTAIRES AVEC PLUSIEURS ÉMETTEURS DE RAYONS X INDIVIDUELS DÉCALÉS

(43) Date of publication of application: 06.03.2024
(73) Proprietor: DENTSPLY SIRONA Inc., York, PA 17401 (US); Sirona Dental Systems GmbH, 64625 Bensheim (DE)
(72) Inventor: Eichner, Stefan, 64625 Bensheim (DE); Maur, Susanne, 64625 Bensheim (DE)
(74) Representative: Venner Shipley LLP

(56) References cited:
- EP-A1- 3 649 957
- EP-A1- 4 011 295
- WO-A1-2020/243383

## Description

### TECHNICAL FIELD OF THE INVENTION

The present invention relates to an x-ray system for producing orthopantomography images in the dental field.

### BACKGROUND OF THE INVENTION

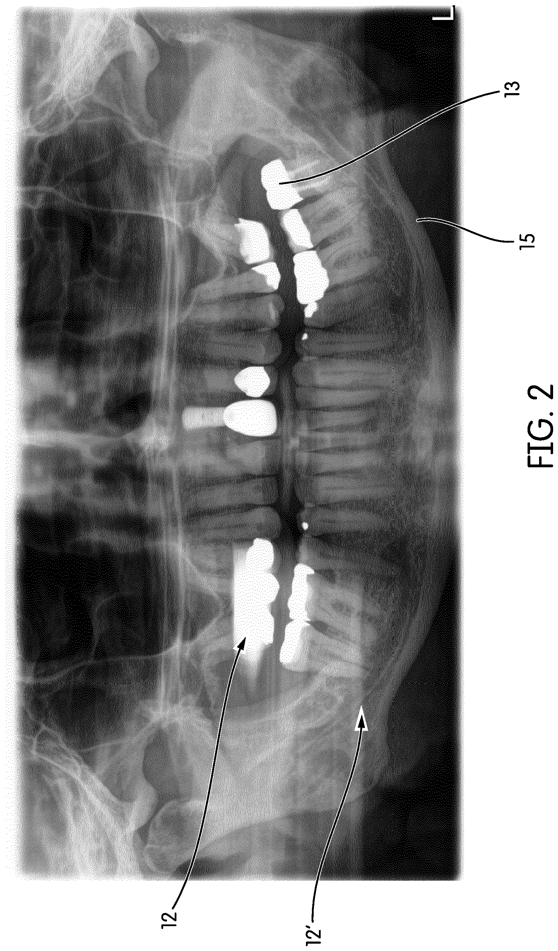

During an orthopantomography or panoramic imaging (PAN), the x-ray emitter and the x-ray detector of an extraoral x-ray system generally move around a patient's head in defined relative positions to each other. This can lead to unfavorable superimposition of the imaged structures, which can complicate the diagnosis and may require repeat exposures. In PAN, movement around the patient's head during imaging results in horizontal blurring of structures outside the sharp layer. The sharp layer defines a layer that preferably runs along the jaw arch, and which has the dental structures to be imaged, and is preferably displayed with good image quality in the panoramic image. For the production of the panoramic image, for example, a "Shift & Add" procedure can be used which sums the projection images of the exposure. Nevertheless, structures lying in the x-ray path create shadowing in the sharp layer and reduce the contrast in the panoramic image. For example, fillings in the current pivot point of the x-ray emitter and x-ray detector unit lead to strong artifacts in the opposing jaw (see example in Fig. 2). These are commonly referred to as opposing jaw artifacts in the prior art. For example, the tooth crown (13) causes an opposing jaw artifact (12) in the representation of the opposite side of the jaw in the panoramic image. Similarly, the jawbone (15), for example, causes an opposing jaw artifact (12') in the representation of the opposing jaw side in the panoramic image.

In the prior art, this problem is mitigated as follows: A different imaging program of the extraoral x-ray system is selected to apply a different x-ray device trajectory for the panoramic imaging. Alternatively, the position and/or orientation of the patient's head in the x-ray device is adjusted to reduce the superimposition of interfering structures and thus opposing jaw artifacts.

In the prior art, the problem cannot be adequately solved, and thus not all artifacts can be reduced. Repeat exposures may be required, resulting in increased radiation exposure to the patient.

In addition, the prior art approach to patient re-positioning and/or imaging program reselection results in complicated and error-prone x-ray device operation and potentially reduced image quality, so that this approach requires a great deal of experience on the part of the x-ray device operator.

EP3649957A1 and EP3711672B respectively disclose devices and methods for processing a panoramic image, wherein opposing jaw artifacts are reduced by software.

### DISCLOSURE OF THE INVENTION

The inventors are currently not aware of any prior art method that enables the reduction of opposing jaw artifacts by vertically blurring the structures outside the sharp layer. Whereby the vertical blurring causes a vertical unsharpness of the structures to be imaged.

An objective of the present invention is to provide a PAN-enabled X-ray system that allows reduction of opposing jaw artifacts by vertically blurring structures outside the sharp layer.

This objective has been achieved by the PAN-enabled X-ray system according to claim 1. The subject-matter of the dependent claims relate to preferred embodiments or further developments.

The invention provides an extraoral x-ray system suitable for a panoramic dental imaging procedure of a patient. The extraoral x-ray system comprises: an x-ray emitter array having at least two individual x-ray emitters each for emitting x-rays, which are offset at least along a predetermined direction (e.g., a patient longitudinal direction or a height / vertical direction); and an x-ray detector for at least partially detecting the x-ray radiations emitted by the individual x-ray emitters during one revolution, wherein the x-ray emitter array and the x-ray detector are disposed movably relative to one another about an axis running parallel to the said predetermined direction, wherein the areas of the x-ray detector which are respectively irradiated by the individual x-ray emitters at least partially overlap; an aperture means for collimating the x-ray radiations emitted by the individual x-ray emitters onto the respective areas to be irradiated; a control device for moving the x-ray emitter array and the x-ray detector about the parallel running axis, and for driving the individual x-ray emitters and for reading out the image series of the respectively irradiated areas of the x-ray detector during the said revolution; a computing unit for generating one panoramic image of a layer to be sharply imaged by using the at least two said image series, wherein the computing unit is configured in such a way that a weighting of image pixels is applied when generating the said one panoramic image, wherein the said image pixels belong at least to the two image series.

An advantageous effect of the present invention is that the offset or height-shifted single emitters result in different transmission angles, so that the corresponding detector signals enable the separation of the overlapping structures in the reconstructed image. In particular, due to the multiple available viewing angles of the height-shifted single emitters, an improved representation of the structures within the sharp layer can be achieved, mainly because the structures outside the sharp layer can be mapped to different positions in the reconstructed panoramic image and can be blurred more. Weighting the image pixels during the reconstruction of the panoramic image can additionally increase the contrast of the structures inside the sharp layer and additionally decrease the contrast of the structures outside the sharp layer. This allows for improved visualization of smaller areas such as caries, lesions, roots, or nerve canals, and diagnostic discoverability. Also, by weighting the image pixels, those structures can be specifically suppressed which cause strong opposing jaw artifacts. The resulting reduction of opposing jaw artifacts in the panoramic image enables simplified x-ray device operation, as possible opposing jaw artifacts no longer need to be taken into account when positioning the patient and selecting the x-ray device trajectory and imaging program. This also allows the use of more suitable transmission angles and thus also contributes to improved image quality. For example, in the area of the anterior teeth, this enables significantly improved imaging, especially in the case of unfavorable tooth positions, such as a large difference in the tooth axes between the upper and lower jaws. The present invention also makes it possible to determine whether imaged structures lie inside or outside the sharp layer. For this purpose, image pixels of the multiple individual x-ray emitters can be compared. If these show different absorption in the reconstructed panoramic image, they are images of structures outside the sharp layer.

### BRIEF DESCRIPTION OF THE DRAWINGS

In the following description, the present invention will be explained in more detail by means of exemplary embodiments and with reference to the drawings, wherein
Fig. 1 - shows an extraoral x-ray system according to one embodiment of the present invention;
Fig. 2 - shows an example image of strong opposing jaw artifacts according to the state of the art;
Figs. 3a, 3b - show two panoramic images, each reconstructed from the two image series of the two single x-ray emitters with different positions and characteristics of the opposing jaw artifacts;
Fig. 4 - shows a schematic detailed representation of the setup of the x-ray emitter array with locally distributed individual x-ray emitters in the extraoral X-ray system from Fig.1.

The reference numbers shown in the drawings designate the elements listed below, which are referred to in the following description of the exemplary embodiments.
1. Extraoral X-ray system
2. X-ray device
3. X-Ray Array
3a,3b,3c. Single x-ray emitter
4. X-ray detector
5. Operating unit
6. Head fixation
7. Bite block
8. Computer
9. Display
10. Patient head
11. Spotlight cone
12. 12', 12", 12" Counter jaw artifact
13. Tooth crown
14. Earring
15. 15' Jaw bone
16. Dental panoramic image

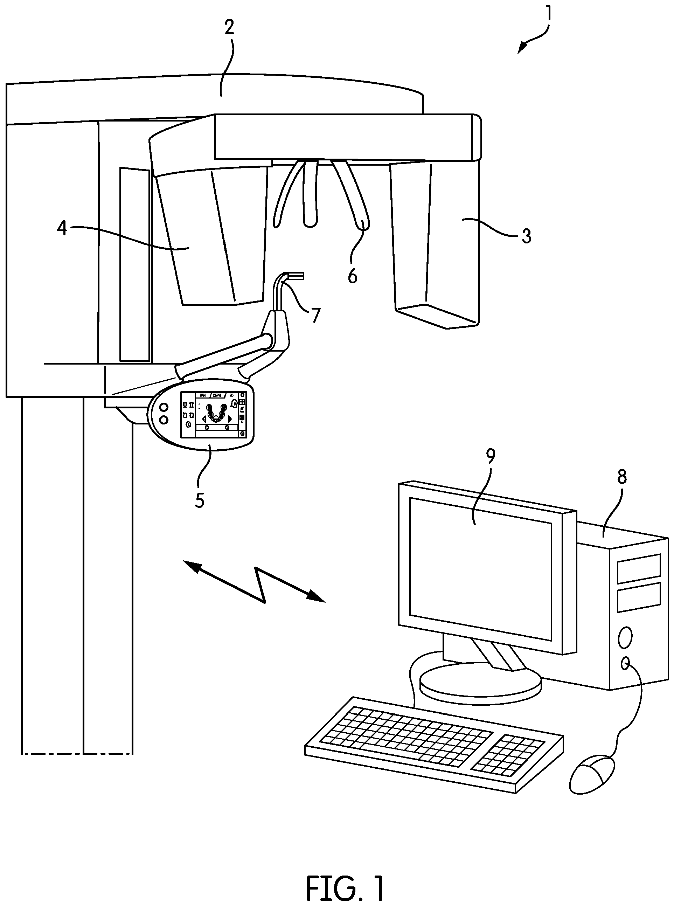

Fig. 1 shows an embodiment of the computer-implemented extraoral x-ray system (1). The method according to the invention is a computer-implemented method, and can be executed on a computer-implemented extraoral x-ray system (1). The method according to the invention is implemented by a computer program comprising computer-readable code. The computer program may be provided on a data storage device. As shown in Fig. 1, the computerized extraoral X-ray system (1) comprises an x-ray device (2) for performing the patient imaging. Prior to the exposure, the patient head is preferably positioned in the x-ray device (2) with the bite block (7) and the head fixation (6). As shown in Fig. 1, the computerized extraoral X-ray system (1) has an operating unit (5), preferably a separate computer (8) or computing unit that can be connected to the x-ray device (2), and preferably a separate display (9), inter alia to visualize data sets. The computer (8) can be connected to the x-ray device (2) via a local network (not shown) or alternatively via the Internet. The computer (8) may be part of a cloud. Alternatively, the computer (8) may be integrated into the x-ray device (2). The calculations can take place in the computer (8) or in the cloud. For this purpose, the raw data can be transmitted in compressed form. The computer (8) executes the computer program and provides the data sets, including for the visualization on the display (9). The display (9) can be spatially separated from the x-ray device (2). Preferably, the computer (8) can also control the x-ray device (2). Alternatively, separate computers (8) may be used for the control and image processing. According to the present invention, the data sets generated by the above embodiment may be presented to a physician for visualization, in particular for diagnostic purposes, preferably by means of the display (9) or a printout.

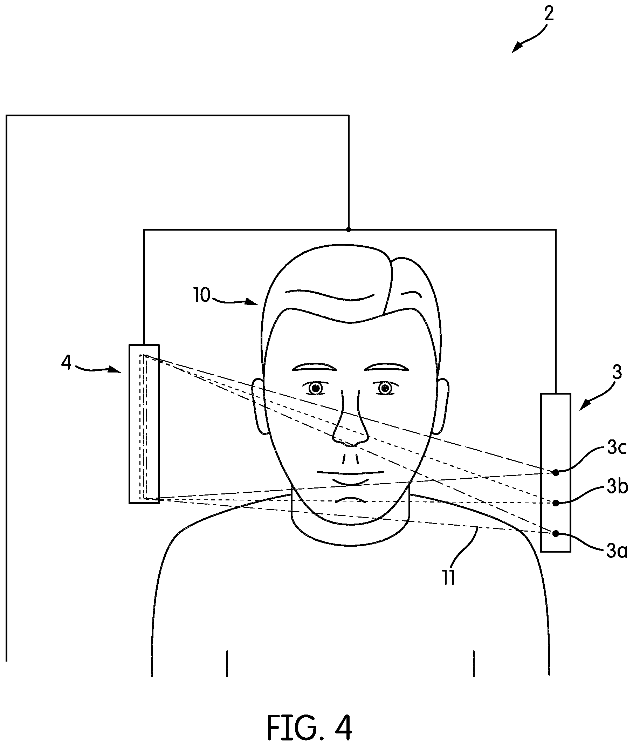

The extraoral x-ray system (1) is suitable for performing the dental panoramic imaging method. As shown in Fig. 1, the x-ray system (1) comprises an x-ray emitter array (3) having at least two individual x-ray emitters (3a;3b;3c) each for emitting x-ray radiation (see Fig. 4), which are offset at least along a predetermined direction (e.g., in the height direction or longitudinal direction of the patient). The x-ray system (1) further comprises an x-ray detector (4) for at least partially detecting the x-ray radiations emitted by the individual x-ray emitters (3a;3b;3c) during one revolution, wherein the x-ray emitter array (3) and the x-ray detector (4) are arranged movably relative to one another about an axis running parallel to said predetermined direction, wherein the areas of the x-ray detector (4) respectively irradiated by the individual x-ray emitters (3a;3b;3c) at least partially overlap. The x-ray system (1) further comprises an aperture means (not shown) for collimating the x-ray radiation emitted by the individual x-ray emitters (3a;3b;3c) onto the said respective areas to be irradiated. The x-ray system (1) further comprises a control device (not shown) for moving the x-ray emitter array (3) and the X-ray detector (4) around the parallel axis, and for controlling the individual x-ray emitters (3a;3b;3c) and for reading out the image series of the respective irradiated surfaces of the X-ray detector (4) during the rotation. The x-ray system (1) further comprises a computing unit (8) for generating one panoramic image (16) of a layer to be sharply imaged by using the at least two said image series, wherein the computing unit is configured (programmed) to apply a weighting of image pixels when generating the said one panoramic image (16), wherein the said image pixels belong to at least the two image series.

In an advantageous embodiment, the total height of the irradiated areas along the predetermined direction is greater than the total width of the irradiated areas along a direction orthogonal thereto by a factor of at least F=3 times. In a further advantageous embodiment, the factor F is in the range 15 to 25, preferably about 22. The total height of the irradiated areas and the total width of the irradiated areas correspond to the effective height and width on the x-ray detector. Whereby the areas irradiated by each of the individual emitters may overlap in height and/or width. Whereby the areas respectively irradiated by the individual emitters can preferably partially overlap in height and can preferably almost completely overlap in width. The factor F leads to a smaller size of the irradiated total area while the height remains the same. This has the advantage of enabling a high readout rate of the x-ray detector with low motion blurring, as well as causing a low proportion of measured scattered radiation on the x-ray detector, which improves the image quality. The lower irradiated area also reduces the applied dose to the patient.

During one revolution, the x-ray emitter array and x-ray detector move around the patient, with the x-ray emitter array emitting x-rays and the x-ray detector detecting the emitted x-rays. For each individual x-ray emitter of the x-ray emitter array, a series of images can be generated during the revolution from the detected x-rays.

In a further embodiment, the operating state of the x-ray emitter array (or the x-ray device) is varied during the revolution such that the position of one of the focal points varies along the predetermined direction. Where the focal points describe the locations within the individual emitters where the x-rays are emitted. The variation of the position of the focal point causes a variation of the transmission direction. Thereby, for example, a different transmission angle can be realized in the anterior region than in the molar region. Preferably, a lower focal point is used in the molar region than in the anterior region. The lower focal point in the molar region serves to reduce opposing jaw artifacts. A higher focal point in the anterior region improves the vertical transmission angles, especially in the case of obliquely positioned teeth, and thus leads to an improvement in image quality and diagnosability.

The dental panoramic image could also be generated by a bitewing or temporomandibular joint acquisition, among others.

In one embodiment, the aperture means comprises an aperture per individual x-ray emitter, which can collimate the x-ray beams of the individual x-ray emitters individually. In another embodiment, the aperture means comprises an aperture that can collimate the X-ray beams of all or several individual emitters.

In another embodiment, the aperture means comprises a mechanical aperture that can vary the aperture opening. This can be used to vary the aperture opening during revolution, for example to increase the aperture opening in the anterior tooth region to enhance blurring of structures outside the sharp layer.

In another embodiment, the aperture means comprises a fixed diaphragm.

The emitter array consists of several small, quickly switchable individual emitters, which are spatially distributed. Emitter arrays can be realized using, among others, carbon nanotubes or cold cathode elements as single emitters. This allows the creation of x-ray projections from different projection angles without x-ray-device movement.

The control device can be designed so that the emitted x-ray beams of the at least two individual x-ray emitters differ in the intensity and/or spectral distribution of the x-ray beams in order to produce an intensity and/or spectral distribution that can be varied along the predetermined direction. The extraoral X-ray system can be an X-ray system which can generate panoramic images only, or which can also generate additional DVT images and/or Ceph images.

To generate the panoramic image, the pixels of the panoramic image are calculated from the image pixels of the image series measured by the x-ray detector. The generation of the panoramic image is usually done by summing up the image pixels. This creates the effect that structures in one layer in space are sharply imaged and structures outside this layer are imaged less sharply. By using the two image series under weighting, this effect is enhanced. The weight of an image pixel can also be zero, which corresponds to the omission of the image information of the image pixel.

The generation of the panoramic image is usually done using time stamps, which allow the association between the detector signals and the single emitters used to determine the focus positions and the beam parameters.

The generation of the panoramic image is usually done by using all angle and height information. This offers advantages for the measurement functionalities within the panoramic image, such as improved depth estimation or position determination of structures, as well as the dimensional measurement of specific regions such as caries, lesions and roots. In addition, advantages arise in the visualization of specific regions, such as a layer representation adapted to an occlusal surface, caries, lesion, nerve canal or root.

In a further advantageous embodiment, the computing unit is configured such that the weighting of the image pixels to be applied increases the contrast of the structures to be imaged in a sharp layer in the panoramic image and/or decreases the contrast of the structures to be imaged outside the sharp layer in the panoramic image. A high contrast results from a high relative brightness difference of the imaged structures and clearly defined contours. This has the beneficial effect of improving the representation of the structures to be imaged within the sharp layer of the panoramic image, thus simplifying diagnosis.

In a further advantageous embodiment, the computing unit is configured in such a way that the weighting to be applied is determined relatively lower for image pixels which exhibit a relatively strong absorption of x-rays outside the sharp layer. The mentioned relatively strong absorption can, for example, be attributed to metals in the opposing jaw. Whether a structure is located in the sharp layer can be determined by comparing at least two preliminary panoramic images, because structures in the sharp layer are mapped to a similar position in the preliminary panoramic images. The preliminary panoramic images will be explained in detail in the subsequent description. A low signal at the detector indicates strong absorption. This embodiment has the beneficial effect of improving the representation of the structures to be imaged within the sharp layer of the panoramic image, thus simplifying diagnosis.

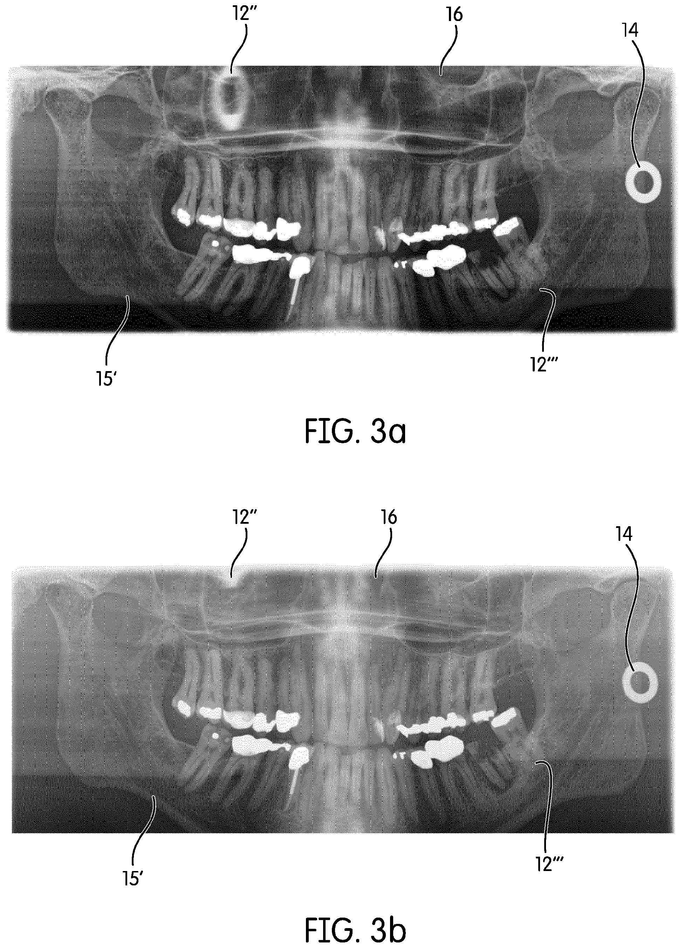

Figs. 3a and 3b show two preliminary panoramic images (16), respectively reconstructed from series of images from single emitters with different focal heights, showing different positions and characteristics of the opposing jaw artifacts (12", 12"'). The earring (14) is mapped as an opposing jaw artifact (12") to a comparatively lower position in Fig. 3a than in Fig. 3b. Similarly, the jawbone (15') is mapped as an opposing jaw artifact (12"') in Fig. 3a (see horizontal running shadow transition) to a comparatively lower position than in Fig. 3b. In contrast thereto, the image of the dental structures (e.g., teeth) of the sharp layer remains at the same position in both preliminary panoramic images (16).

In a further advantageous embodiment, the computing unit is configured such that the weighting to be applied is determined to be relatively lower for image pixels which have relatively large differences in absorption of the x-rays along the predetermined direction outside the sharp layer. This is advantageous, for example, in the case of large signal differences at a small local distance, for example to suppress artifacts of the jawbone of the opposing jaw. This has the beneficial effect of improving the representation of the structures to be imaged within the sharp layer of the panoramic image, thus simplifying diagnosis.

In a further advantageous embodiment, the computing unit is configured such that the weighting to be applied is determined on the basis of preliminary panoramic images, each of which is generated from one of the at least two image series and depicts the sharp layer. The generation of the panoramic image can also be realized by weighted computation, for example summation, of the two preliminary panoramic images. This has the advantage that the representation of the structures to be imaged within the sharp layer of the panoramic image is improved compared to the representation in the preliminary panoramic images.

In a further advantageous embodiment, the computing unit is configured such that the weighting to be applied is determined relatively lower for the image pixels which have a relatively different absorption of the x-rays in the corresponding pixels or their local neighborhood of the preliminary panoramic images. If there is a relatively different absorption of the x-rays in the corresponding pixels or their local neighborhood of the preliminary panoramic images, these structures outside the sharp layer are mapped in the panoramic image. Their low weighting has the advantageous effect of an improved representation of the structures to be imaged within the sharp layer of the panoramic image and thus simplifies the diagnosis.

In another advantageous embodiment, the computing unit is configured to determine the applicable weighting of image pixels based on knowledge of the patient anatomy and x-ray device geometry and x-ray device motion retrieved from a storage device. This has the beneficial effect of improving discrimination between structures outside and inside the sharp layer. Thus, this leads to an improved representation of the structures to be imaged within the sharp layer of the panoramic image and simplifies diagnosis.

In a further advantageous embodiment, the computing unit is configured to align the position/shape of the sharp layer to be imaged with the patient's dental structures such as teeth and jaw bones, tooth crowns, tooth roots, tooth root tips, periodontal crevices, nerve canals of jaws and teeth, jaw joints, or dental pathologies of the patient such as caries and inflammations. For the aforementioned alignment of the sharp layer to be imaged, common image optimization methods can be used, including the so-called autofocus function, by calculating it from a series of images, or by calculating it separately for each series of images and then calculating the separate autofocus results together, or by calculating it jointly from the at least two series of images. This has the beneficial effect of improving the representation of the relevant dental structures within the sharp layer of the panoramic image and thus simplifies diagnosis.

In another advantageous embodiment, the computing unit is configured to align or realign the position/shape of the sharp slice to be imaged to the patient's dental structures such as teeth and jaw bones, tooth crowns, tooth roots, tooth root tips, periodontal crevices, nerve canals of jaw and teeth, occlusal surfaces, temporomandibular joints, or dental pathologies of the patient such as caries and inflammations, by using the weighting of the image pixels from at least two image series. In the aforementioned determination of the sharp layer to be imaged, the autofocus function can be used. This has the beneficial effect of improving the visualization of relevant dental structures within the sharp layer of the panoramic image, thus simplifying diagnosis. By using several weighted image series with different projection angles, this leads to an advantageous representation of inclined layers in the panoramic image, such as the representation of occlusal surfaces.

In a further embodiment, multiple panoramic images or partial areas of the panoramic images are generated for which the position of the sharp layer varies in a predetermined area, e.g., in the lingual or buccal direction, using the weighting of the image pixels when generating the panoramic images. This offers the user the possibility to navigate in a narrow range, around the sharp layer, e.g. to show individual roots in the molar region. This enables improved visualization and position determination of the dental structures.

In a further advantageous embodiment, the control device controls the x-ray emitter array and the aperture means in such a way that the said areas to be irradiated are sequentially irradiated by the incident x-ray beams of the at least two individual x-ray emitters. This has the advantageous effect that the respective irradiated areas of the x-ray detector can be read out for each focus position as independently as possible.

In a further advantageous embodiment, the control device drives the at least two individual x-ray emitters sequentially. This has the advantageous effect that the areas to be irradiated are sequentially irradiated by the incident X-ray beams of the at least two individual x-ray emitters.

In a further advantageous embodiment, the control device controls by means of the aperture means to sequentially block the x-rays emitted from the at least two individual x-ray emitters so that an image of the image series is predominantly resulting from the x-rays of one of the at least two individual x-ray emitters. This corresponds to a mechanical, so-called shutter aperture, which sequentially covers all except one of the individual x-ray emitters. This has the advantage that the areas to be irradiated are irradiated sequentially by the incident X-ray radiation of the at least two individual x-ray emitters.

## Claims

1. An extraoral x-ray system (1) suitable for dental panoramic imaging of a patient, comprising:
an x-ray emitter array (3) having at least two individual x-ray emitters (3a;3b;3c) each for emitting x-rays, and offset at least along a predetermined direction;
an x-ray detector (4) for at least partially detecting the x-rays emitted from the individual x-ray emitters (3a;3b;3c) during one revolution, wherein the x-ray emitter array (3) and the x-ray detector (4) are arranged for movement relative to each other about an axis running parallel to said predetermined direction, wherein the areas of the x-ray detector (4) respectively irradiated by the individual x-ray emitters (3a;3b;3c) at least partially overlap;
an aperture means for collimating the x-ray radiation emitted by the individual x-ray emitters (3a;3b;3c) onto the respective areas to be irradiated;
a control device for moving the x-ray emitter array (3) and the x-ray detector (4) about the parallel running axis, and for driving the individual x-ray emitters (3a;3b;3c) and for reading out the image series of the respective irradiated areas of the x-ray detector (4) during the said revolution;
a computing unit for generating one panoramic image (16) of a layer to be sharply imaged by using the at least two said image series, wherein the computing unit is configured to apply a weighting of image pixels when generating the said one panoramic image (16), wherein the said image pixels belong to at least the two image series.

2. The extraoral x-ray system (1) according to the preceding claim 1, wherein the computing unit is configured such that the weighting of the image pixels to be applied increases the contrast of the structures to be imaged in the said sharp layer in the panoramic image (16) and/or decreases the contrast of the structures to be imaged outside the said sharp layer in the panoramic image (16).

3. The extraoral x-ray system (1) according to any one of the preceding claims 1 to 2, wherein the computing unit is configured such that the weighting to be applied is determined to be relatively lower for image pixels which have a relatively strong absorption of x-rays outside the said sharp layer.

4. The extraoral x-ray system (1) according to any one of the preceding claims 1 to 3, wherein the computing unit is configured to determine the weighting to be applied relatively lower for image pixels which have relatively strongly different absorptions of the X-rays along the predetermined direction outside the said sharp layer.

5. The extraoral x-ray system (1) according to any one of the preceding claims 1 to 4, wherein the computing unit is configured to determine the weighting to be applied on the basis of preliminary panoramic images, each of which is generated from one of the at least two image series and depicts the sharp layer.

6. The extraoral x-ray system (1) according to the preceding claim 5, wherein the computing unit is configured to determine the weighting to be applied relatively lower for the image pixels having a relatively different absorption of the X-rays in the corresponding pixels or their local neighborhood of the preliminary panoramic images.

7. The extraoral x-ray system (1) according to any one of the preceding claims 1 to 6, wherein the computing unit is configured to determine the weighting to be applied to the image pixels based on knowledge of the patient anatomy and the x-ray system geometry and the x-ray system movement to be retrieved from a storage medium.

8. The extraoral x-ray system (1) according to any one of the preceding claims 1 to 7, wherein the computing unit is configured to align the position of the sharp layer to be imaged with the patient's dental structures such as teeth and jaw bones, tooth crowns, tooth roots, tooth root tips, periodontal crevices, nerve canals of jaws and teeth, temporomandibular joints, or dental pathologies of the patient such as caries and inflammations.

9. The extraoral x-ray system (1) according to any of the preceding claims 1 to 8, wherein the computing unit is configured to adapt by using the weighting of the image pixels from at least two image series, the position of the sharp layer to be imaged to the patient's dental structures such as teeth and jaw bones, tooth crowns, tooth roots, tooth root tips, periodontal gaps, nerve canals of jaws and teeth, occlusal surfaces, jaw joints, or dental pathologies, or dental pathologies of the patient such as caries and inflammations.

10. The extraoral x-ray system (1) according to any one of the preceding claims 1 to 9, wherein a plurality of panoramic images are generated for which the position of the said sharp layer varies in a predetermined range, wherein the weighting of the image pixels is used in the generation of the panoramic images.

11. The extraoral x-ray system (1) according to any one of the preceding claims 1 to 10, wherein the control device, the x-ray emitter array and the aperture means are controlled such that the areas to be irradiated are sequentially irradiated by the incident x-ray beams of the at least two individual x-ray emitters.

12. The extraoral x-ray system (1) according to the preceding claim 11, wherein the control device sequentially drives the at least two individual x-ray emitters.

13. The extraoral x-ray system (1) according to the preceding claim 11, wherein said control means, sequentially blocks by means of said aperture means the X-ray radiations emitted from said at least two individual x-ray emitters so that an image of said image series is predominantly resulting from the x-ray radiation of one of said at least two individual x-ray emitters.

14. Extraoral x-ray system (1) according to the preceding claim 11, wherein the total height of the irradiated areas along the predetermined direction is greater than the total width along a direction orthogonal thereto by a factor (F) of at least 3 times.

## Patentansprüche

1. Extraorales Röntgensystem (1), das für zahnmedizinische Panoramabildgebung eines Patienten geeignet ist, umfassend:
eine Röntgenstrahleranordnung (3), die mindestens zwei einzelne Röntgenstrahler (3a; 3b; 3c) aufweist, die jeweils zum Emittieren von Röntgenstrahlen sind und mindestens entlang einer vorbestimmten Richtung versetzt sind;
einen Röntgendetektor (4) zum zumindest teilweisen Erfassen der von den einzelnen Röntgenstrahlern (3a; 3b; 3c) während einer Umdrehung emittierten Röntgenstrahlen, wobei die Röntgenstrahleranordnung (3) und der Röntgendetektor (4) für eine Bewegung in Bezug aufeinander um eine Achse angeordnet sind, die parallel zu der vorbestimmten Richtung verläuft, wobei sich die Bereiche des Röntgendetektors (4), die jeweils von den einzelnen Röntgenstrahlern (3a; 3b; 3c) bestrahlt werden, zumindest teilweise überlappen;
eine Blendeneinrichtung zum Kollimieren der von den einzelnen Röntgenstrahlern (3a; 3b; 3c) emittierten Röntgenstrahlung auf die jeweiligen zu bestrahlenden Bereiche;
eine Steuervorrichtung zum Bewegen der Röntgenstrahleranordnung (3) und des Röntgendetektors (4) um die parallel verlaufende Achse und zum Ansteuern der einzelnen Röntgenstrahler (3a; 3b; 3c) und zum Auslesen der Bilderreihen der jeweils bestrahlten Bereiche des Röntgendetektors (4) während der Umdrehung;
eine Recheneinheit zum Erzeugen eines Panoramabilds (16) einer scharf abzubildenden Schicht unter Verwendung der mindestens zwei Bilderreihen, wobei die Recheneinheit konfiguriert ist, um beim Erzeugen des einen Panoramabilds (16) eine Gewichtung von Bildpixeln anzuwenden, wobei die Bildpixel zu mindestens den zwei Bilderreihen gehören.

2. Extraorales Röntgensystem (1) nach dem vorhergehenden Anspruch 1, wobei die Recheneinheit konfiguriert ist, sodass die anzuwendende Gewichtung der Bildpixel den Kontrast der abzubildenden Strukturen in der scharfen Schicht in dem Panoramabild (16) erhöht und/oder den Kontrast der abzubildenden Strukturen außerhalb der scharfen Schicht in dem Panoramabild (16) verringert.

3. Extraorales Röntgensystem (1) nach einem der vorhergehenden Ansprüche 1 bis 2, wobei die Recheneinheit konfiguriert ist, sodass die anzuwendende Gewichtung für Bildpixel, die eine relativ starke Absorption von Röntgenstrahlen außerhalb der scharfen Schicht aufweisen, als relativ niedriger bestimmt wird.

4. Extraorales Röntgensystem (1) nach einem der vorhergehenden Ansprüche 1 bis 3, wobei die Recheneinheit konfiguriert ist, um die anzuwendende Gewichtung für Bildpixel, die relativ stark unterschiedliche Absorptionen der Röntgenstrahlen entlang der vorbestimmten Richtung außerhalb der scharfen Schicht aufweisen, relativ niedriger zu bestimmen.

5. Extraorales Röntgensystem (1) nach einem der vorhergehenden Ansprüche 1 bis 4, wobei die Recheneinheit dazu konfiguriert ist, um die anzuwendende Gewichtung auf Grundlage von vorläufigen Panoramabildern zu bestimmen, die jeweils aus einer der mindestens zwei Bilderreihen erzeugt werden und die scharfe Schicht abbilden.

6. Extraorales Röntgensystem (1) nach dem vorhergehenden Anspruch 5, wobei die Recheneinheit konfiguriert ist, um die anzuwendende Gewichtung für die Bildpixel, die eine relativ unterschiedliche Absorption der Röntgenstrahlen in den entsprechenden Pixeln oder ihrer lokalen Nachbarschaft der vorläufigen Panoramabilder aufweisen, relativ niedriger zu bestimmen.

7. Extraorales Röntgensystem (1) nach einem der vorhergehenden Ansprüche 1 bis 6, wobei die Recheneinheit konfiguriert ist, um die auf die Bildpixel anzuwendende Gewichtung auf Grundlage von Kenntnis der Patientenanatomie und der Röntgensystemgeometrie und der aus einem Speichermedium abzurufenden Röntgensystembewegung zu bestimmen.

8. Extraorales Röntgensystem (1) nach einem der vorhergehenden Ansprüche 1 bis 7, wobei die Recheneinheit konfiguriert ist, um die Position der abzubildenden scharfen Schicht auf die dentalen Strukturen des Patienten, wie beispielsweise Zähne und Kieferknochen, Zahnkronen, Zahnwurzeln, Zahnwurzelspitzen, Parodontalspalten, Nervenkanäle von Kiefern und Zähnen, Kiefergelenke oder dentale Pathologien des Patienten, wie beispielsweise Karies und Entzündungen auszurichten.

9. Extraorales Röntgensystem (1) nach einem der vorhergehenden Ansprüche 1 bis 8, wobei die Recheneinheit konfiguriert ist, um unter Verwendung der Gewichtung der Bildpixel aus mindestens zwei Bilderreihen die Position der abzubildenden scharfen Schicht an die dentalen Strukturen des Patienten, wie beispielsweise Zähne und Kieferknochen, Zahnkronen, Zahnwurzeln, Zahnwurzelspitzen, Parodontalspalten, Nervenkanäle von Kiefern und Zähnen, Kauflächen, Kiefergelenke, oder dentale Pathologien, oder dentale Pathologien des Patienten, wie beispielsweise Karies und Entzündungen, anzupassen.

10. Extraorales Röntgensystem (1) nach einem der vorhergehenden Ansprüche 1 bis 9, wobei eine Vielzahl von Panoramabildern erzeugt wird, bei denen die Position der scharfen Schicht in einem vorbestimmten Bereich variiert, wobei die Gewichtung der Bildpixel bei der Erzeugung der Panoramabilder verwendet wird.

11. Extraorales Röntgensystem (1) nach einem der vorhergehenden Ansprüche 1 bis 10, wobei die Steuervorrichtung, die Röntgenstrahleranordnung und die Blendeneinrichtung gesteuert werden, sodass die zu bestrahlenden Bereiche nacheinander von den einfallenden Röntgenstrahlen der mindestens zwei einzelnen Röntgenstrahler bestrahlt werden.

12. Extraorales Röntgensystem (1) nach dem vorhergehenden Anspruch 11, wobei die Steuervorrichtung nacheinander die mindestens zwei einzelnen Röntgenstrahler ansteuert.

13. Extraorales Röntgensystem (1) nach dem vorhergehenden Anspruch 11, wobei die Steuereinrichtung mittels der Blendeneinrichtung die von den mindestens zwei einzelnen Röntgenstrahlern emittierte Röntgenstrahlung nacheinander blockiert, sodass ein Bild der Bilderreihe überwiegend aus der Röntgenstrahlung von einem der mindestens zwei einzelnen Röntgenstrahler resultiert.

14. Extraorales Röntgensystem (1) nach dem vorhergehenden Anspruch 11, wobei die Gesamthöhe der bestrahlten Bereiche entlang der vorbestimmten Richtung um einen Faktor (F) von mindestens dem Dreifachen größer ist als die Gesamtbreite entlang einer dazu orthogonalen Richtung.

## Revendications

1. Système à rayons X extra-buccal (1) approprié pour l'imagerie panoramique dentaire d'un patient, comprenant :
un réseau d'émetteurs de rayons X (3) comportant au moins deux émetteurs de rayons X individuels (3a ; 3b ; 3c) chacun destinés à émettre des rayons X, et décalés au moins le long d'une direction prédéfinie ;
un détecteur de rayons X (4) destiné à détecter au moins partiellement les rayons X émis par les émetteurs de rayons X individuels (3a ; 3b ; 3c) pendant une révolution, ledit réseau d'émetteurs de rayons X (3) et ledit détecteur de rayons X (4) étant agencés pour un déplacement relatif l'un par rapport à l'autre autour d'un axe s'étendant parallèlement à ladite direction prédéfinie, lesdites zones du détecteur de rayons X (4) respectivement irradiées par les émetteurs de rayons X individuels (3a ; 3b ; 3c) se chevauchant au moins partiellement ;
un moyen d'ouverture destiné à collimater le rayonnement de rayons X émis par les émetteurs de rayons X individuels (3a ; 3b ; 3c) sur les zones respectives devant être irradiées ;
un dispositif de commande destiné à déplacer le réseau d'émetteurs de rayons X (3) et le détecteur de rayons X (4) autour de l'axe s'étendant parallèlement, et à commander aux émetteurs de rayons X individuels (3a ; 3b ; 3c) et à lire la série d'images des zones irradiées respectives du détecteur de rayons X (4) pendant ladite révolution ;
une unité de calcul destinée à générer une image panoramique (16) d'une couche devant être imagée de manière nette à l'aide desdites au moins deux desdites séries d'images, ladite unité de calcul étant conçue pour appliquer une pondération de pixels d'image lors de la génération de ladite une image panoramique (16), lesdits pixels d'image appartenant à au moins les deux séries d'images.

2. Système à rayons X extra-buccal (1) selon la revendication 1 précédente, ladite unité de calcul étant conçue de sorte que la pondération des pixels d'image devant être appliquée augmente le contraste des structures devant être imagées dans ladite couche nette dans l'image panoramique (16) et/ou diminue le contraste des structures devant être imagées à l'extérieur de ladite couche nette dans l'image panoramique (16).

3. Système à rayons X extra-buccal (1) selon l'une quelconque des revendications 1 à 2 précédentes, ladite unité de calcul étant conçue de sorte que la pondération devant être appliquée soit déterminée de manière à étant relativement plus faible pour les pixels d'image qui présentent une absorption relativement forte des rayons X à l'extérieur de ladite couche nette.

4. Système à rayons X extra-buccal (1) selon l'une quelconque des revendications 1 à 3 précédentes, ladite unité de calcul étant conçue pour déterminer la pondération devant être appliquée relativement plus faible pour des pixels d'image qui présentent des absorptions des rayons X relativement fortement différentes le long de la direction prédéfinie à l'extérieur de ladite couche nette.

5. Système à rayons X extra-buccal (1) selon l'une quelconque des revendications 1 à 4 précédentes, ladite unité de calcul étant conçue pour déterminer la pondération devant être appliquée sur la base d'images panoramiques préliminaires, chacune d'elles étant générée à partir de l'une desdites au moins deux séries d'images et représentant la couche nette.

6. Système à rayons X extra-buccal (1) selon la revendication 5 précédente, ladite unité de calcul étant conçue pour déterminer la pondération devant être appliquée relativement plus faible pour les pixels d'image présentant une absorption relativement différente des rayons X dans les pixels correspondants ou leur voisinage local avec les images panoramiques préliminaires.

7. Système à rayons X extra-buccal (1) selon l'une quelconque des revendications 1 à 6 précédentes, ladite unité de calcul étant conçue pour déterminer la pondération devant être appliquée aux pixels d'image sur la base de la connaissance de l'anatomie du patient et de la géométrie du système à rayons X et du déplacement du système à rayons X devant être extrait d'un support de stockage.

8. Système à rayons X extra-buccal (1) selon l'une quelconque des revendications 1 à 7 précédentes, ladite unité de calcul étant conçue pour aligner la position de la couche nette devant être imagée avec les structures dentaires du patient telles que les dents et les os de la mâchoire, les couronnes dentaires, les racines dentaires, les extrémités des racines dentaires, les crevasses parodontales, les canaux nerveux des mâchoires et des dents, les articulations temporo-mandibulaires ou les pathologies dentaires du patient telles que les caries et les inflammations.

9. Système à rayons X extra-buccal (1) selon l'une quelconque des revendications 1 à 8 précédentes, ladite unité de calcul étant conçue pour s'adapter en utilisant la pondération des pixels d'image provenant d'au moins deux séries d'images, ladite position de la couche nette devant être imagée sur les structures dentaires du patient telles que les dents et les os de la mâchoire, les couronnes dentaires, les racines dentaires, les extrémités des racines dentaires, les espaces parodontaux, les canaux nerveux des mâchoires et des dents, les surfaces occlusales, les articulations de la mâchoire ou les pathologies dentaires, ou les pathologies dentaires du patient telles que les caries et les inflammations.

10. Système à rayons X extra-buccal (1) selon l'une quelconque des revendications 1 à 9 précédentes, une pluralité d'images panoramiques étant générées pour lesquelles la position de ladite couche nette varie dans une plage prédéfinie, ladite pondération des pixels d'image étant utilisée dans la génération des images panoramiques.

11. Système à rayons X extra-buccal (1) selon l'une quelconque des revendications 1 à 10 précédentes, ledit dispositif de commande, ledit réseau d'émetteurs de rayons X et ledit moyen d'ouverture étant commandés de sorte que les zones devant être irradiées soient irradiées de manière séquentielle par les faisceaux de rayons X incidents desdits au moins deux émetteurs de rayons X individuels.

12. Système à rayons X extra-buccal (1) selon la revendication 11 précédente, ledit dispositif de commande commandant de façon séquentielle lesdits au moins deux émetteurs de rayons X individuels.

13. Système à rayons X extra-buccal (1) selon la revendication 11 précédente, ledit moyen de commande bloquant de façon séquentielle au moyen dudit moyen d'ouverture les rayonnements de rayons X émis par lesdits au moins deux émetteurs de rayons X individuels de sorte qu'une image de ladite série d'images résulte principalement du rayonnement de rayons X de l'un desdits au moins deux émetteurs de rayons X individuels.

14. Système à rayons X extra-buccal (1) selon la revendication 11 précédente, ladite hauteur totale des zones irradiées le long de la direction prédéfinie étant supérieure à la largeur totale le long d'une direction orthogonale à celle-ci d'un facteur (F) d'au moins 3.
